# EUROPÄISCHE PATENTSCHRIFT

(11) **EP 2 117 418 B1**
(45) Veröffentlichungstag und Bekanntmachung des Hinweises auf die Patenterteilung: **27.01.2016**
(21) Anmeldenummer: 08709074.2
(22) Anmeldetag: 18.02.2008
(51) Int. Cl.: A61B 5/00, A61B 3/11, A61F 2/16, A61F 9/00

(54) **IMPLANTIERBARES SYSTEM ZUR BESTIMMUNG DES AKKOMMODATIONSBEDARFES DURCH OPTISCHE MESSUNG DES PUPILLENDURCHMESSERS UND DER UMFELDLEUCHTDICHTE**
IMPLANTABLE SYSTEM FOR DETERMINING THE ACCOMMODATION REQUIREMENT BY OPTICAL MEASUREMENT OF THE PUPIL DIAMETER AND THE SURROUNDING LUMINANCE
SYSTÈME IMPLANTABLE POUR DÉTERMINER LE BESOIN D'ACCOMMODATION PAR MESURE OPTIQUE DU DIAMÈTRE DE LA PUPILLE ET DE LA LUMINANCE AMBIANTE

(30) Priorität: 21.02.2007 DE 102007008375
(43) Veröffentlichungstag der Anmeldung: 18.11.2009
(73) Patentinhaber: Karlsruher Institut für Technologie, 76131 Karlsruhe (DE); Universität Rostock, 18051 Rostock (DE)
(72) Erfinder: KLINK, Simon, 70329 Stuttgart (DE); BRETTHAUER, Georg, 76139 Karlsruhe (DE); GUTHOFF, Rudolf, 18119 Rostock (DE); GENGENBACH, Ulrich, 75196 Remchingen (DE); BERGEMANN, Mark, 73033 Göppingen (DE); KOKER, Torsten, 76297 Stutensee (DE); RÜCKERT, Wolfgang, 42489 Wülfrath (DE)
(74) Vertreter: Fitzner, Uwe
(86) Internationale Anmeldenummer: PCT/EP2008/051937
(87) Internationale Veröffentlichungsnummer: WO 2008/101897

(56) Entgegenhaltungen:
- WO-A-02/085245
- WO-A-2004/073547
- CA-A1- 2 313 693
- US-A- 6 139 577

## Beschreibung

Die vorliegende Anmeldung nimmt die Priorität der 10 2007 008 375.2-55 in Anspruch.

Gegenstand der Erfindung ist ein implantierbares System zur Bestimmung des Akkommodationsbedarfes in einem künstlichen Akkommodationssystem durch optische Messung des Pupillendurchmessers und der Umfeldleuchtdichte und dessen Verwendung zur Wiederherstellung der Akkommodationsfähigkeit.

Das menschliche Auge ist ein optisches System, das mit Hilfe mehrerer lichtbrechender Grenzflächen Objekte scharf auf der Netzhaut (retina) abbildet. Hierbei passieren die Lichtwellen die Hornhaut (cornea), das Kammerwasser in der Vorderkammer (camera anterior bulbi), die Linse (lens crystallina) und den Glaskörper in der Hinterkammer (camera vitrea bulbi), die alle unterschiedliche Brechungsindizes aufweisen. Ändert sich die Gegenstandsweite des betrachteten Objektes, ist es für eine Abbildung mit gleich bleibender Schärfe auf der Netzhaut notwendig, dass sich das Abbildungsverhalten des optischen Systems ändert. Beim menschlichen Auge wird dies durch eine Verformung der Linse mit Hilfe des Ziliarmuskels (musculus ciliaris) realisiert, wodurch sich im Wesentlichen die Form und die Lage der Linsenvorder- und -rückseite ändern (Akkommodation). Bei einem intakten Akkommodationssystem eines jugendlichen Menschen kann so die Scheitelbrechkraft des Systems zwischen Ferneinstellung (desakkommodierter Zustand) und Naheinstellung (akkommodierter Zustand) um 14 dpt (Akkommodationsbreite) verändert werden. Dadurch können bei einem normalsichtigen (emmetropen) jugendlichen Menschen Objekte, die sich zwischen dem im Unendlichen liegenden Fernpunkt und dem sich in etwa 7 cm vor der Hornhaut liegenden Nahpunkt befinden, scharf auf der Netzhaut abgebildet werden.

Da die Fähigkeit des menschlichen Auges zur Akkommodation mit zunehmendem Alter abnimmt, sind eine Anzahl von künstlich implantierbaren Linsensystemen mit variabler Brennweite entwickelt worden.

Bei potentiell akkommodierenden Intraokularlinsen handelt es sich um Linsen oder Linsensysteme, die nach operativer Entfernung der natürlichen Linse anstelle dieser eingesetzt und vorwiegend im Kapselsack befestigt werden. Durch eine noch vorhandene, jedoch geringe Restkontraktion des Ziliarmuskels, soll über eine Haptik eine axiale Verschiebung der Linse erreicht werden.

Vorrichtungen zur Wiederherstellung der Akkommodationsfähigkeit sind beispielsweise aus der DE 101 55 345 C2, US 66 38 304 B2, WO 03/017873 A1 und US 4373218, DE 94 22 429 U1, DE 201 11 320 U1, DE 100 62 218 A1, DE 10139027, WO 02/083033, DE 10125829 A1, US 2004/0181279A1, US2002/0149743, US 6120538, US 6120538, DE 10155345 C2, US6096078, US6638304 , US6638304 und WO004605 bekannt.

Ferner gibt es zahlreiche wissenschaftliche Veröffentlichungen zum Thema Akkommodationsfähigkeit von Linsensystemen. Beispielhaft sei auf folgende Veröffentlichungen verwiesen:
Schneider, H.; Stachs, O.; Guthoff, R.: Evidenzbasierte Betrachtungen zu akkommodativen Kunstlinsen. 102. Jahrestagung der Deutschen Ophthalmologischen Gesellschaft (Berlin, Germany, September 23rd-26th 2004) (2004)); (Kammann, J.; Dornbach, G.: Empirical results regarding accommodative lenses. In: Current Aspects of Human Accommodation. Hrsg.: Guthoff, R.; Ludwig, K. Kaden Verlag Heidelberg (2001) 163-170, Fine, H.; Packer M.; Hoffmann R.: Technology generates IOL with amplitude of accommodation" (Ophtalmology Times Special Report, March 15th 2005) (2005), Lavin, M.: Multifocal intraocular lenses - part 1. Optometry Today 5/2001 (2001) 34-37; Lavin, M.: Multifocal intraocular lenses - part 2. Optometry Today 8/2001 (2001) 43-44. Nishi, O.; Nishi, K.; Mano, C.; Ichihara, M.; Honda, T.: Controlling the capsular shape in lens refilling. Archives of Ophthalmology 115(4) (1997) 507-510; Fine, I.H.: The SmartLens- a fabulous new IOL technology. Eye World 7(10) (2002).

Insgesamt ist festzustellen, dass grundsätzlich die im Rahmen einer Kataraktextraktion implantierte Kunstlinse nicht in der Lage ist, auf unterschiedliche Entfernungen zu fokussieren. Das Problem, daß ab einem Alter von ca. 45 Jahren die Fähigkeit des menschlichen Auges, auf einen Leseabstand von ca. 30 cm ausreichend zu Akkommodieren verloren gegangen ist, wird mithin durch solche Operationen auch nicht gelöst. Bisherige Versuche, intraokulare Strukturen, insbesondere die Ziliarmuskelaktivität zur mechanischen Brechkraftänderung implantierbarer Systeme zu nutzen, sind aus biologischen Gründen bisher nicht gelungen. Mittelfristig ist dies auch nicht zu erwarten.

Ein Weg den Akkommodationsbedarf zu ermitteln, ist die Messung des Pupillendurchmessers und der Umfeldleuchtdichte. Aus der US 6638304 B2 sind Möglichkeiten zur Messung von Leuchtdichte und Pupillendurchmesser bekannt. Dabei werden die Leuchtdichte mit einem Fotosensor und der Pupillendurchmesser mit einer Elektrode gemessen, welche Potenzialänderungen der Iris detektiert.

Ferner sind für die optische Messung des Pupillendurchmessers sogenannte Pupillometer bekannt. Diese senden meist Infrarotstrahlung aus und detektieren das reflektierte Licht, woraus der Pupillendurchmesser geschätzt werden kann.

Die bisher zur Verfügung stehenden Pupillometer sind jedoch groß und schwer. Sie sind daher für eine Implantation völlig ungeeignet. Außerdem benötigt die Bildverarbeitung zur Detektion und zur Messung der Pupille eine hohe Rechenleistung.

Die Verwendung einer Elektrode im Irismuskel bringt Unsicherheiten bezüglich der Gewebeveränderungen mit sich. Wird die Elektrode vom Gewebe umkapselt, ist kein ausreichendes Signal mehr messbar. Außerdem erfordert dies einen zusätzlichen Aufwand und ein bisher unvorhersehbares Risiko bei der Implantation.

Davon ausgehend ist es Aufgabe der vorliegenden Erfindung, ein implantierbares Akkommodationssystem vorzuschlagen, das in den Kapselsack implantiert wird und seine Steuerimpulse unabhängig von der Ziliarkörperaktivität gewinnt.

Diese Aufgabe wird durch ein implantierbares System zur Bestimmung des Akkommodationsbedarfs in einem künstlichen Akkommodationssystem durch optische Messung des Pupillendurchmessers und der Umfeldleuchtdichte gelöst umfassend
a) wenigstens ein optisches System,
b) wenigstens ein zum Ziliarmuskel berührungsloses Informationserfassungssystem mit Mitteln zur Messung einer Pupillenweite und einer Leuchtdichte mindestens an einem Auge als körpereigenes Steuersignal für den Akkommodationsbedarf,
c) wenigstens ein Informationsverarbeitungssystem zwecks Erzeugung eines Stellsignals für das optische System aus den erfassten körpereigenen Steuersignalen oder zwecks Umschaltung in den Stand-by Modus
d) wenigstens ein Energieversorgungssystem und
e) wenigstens ein Befestigungssystem.
wobei das System einen Sensor oder mehrere Sensoren mit Sensorelementen zur optischen Messung des Pupillendurchmessers und der Umfeldleuchtdichte aufweist.

Ein solches Akkommodationssystem mit den Merkmalen a) - e) ist beispielsweise in der nicht vorveröffentlichten Deutschen Patentanmeldung 102005038542 beschrieben. Danach sind die einzelnen Systeme zu einem, bzw. in mehreren Regelkreisen verschaltet. Das optische System, das Informationserfassungssystem, das Informationsverarbeitungssystem, das Energieversorgungssystem und das Befestigungssystem sind vorzugsweise zu einem Implantat zusammengefasst, welches zur Wiederherstellung der Akkommodationsfähigkeit des tierischen oder menschlichen Auges in dieses mittels des Befestigungssystems einsetzbar ist. Hierbei ist das optische System im Strahlengang des Auges angeordnet und bildet mit diesem zusammen den dioptrischen Apparat des Auges. In gleicher Weise sind vorzugsweise das Informationserfassungssystem, das Informationsverarbeitungssystem und das Energieversorgungssystem außerhalb des Strahlengangs angeordnet.

Das optische System, bestehend aus einem oder mehreren aktiv-optischen Elementen und / oder einer oder mehreren von Aktoren axial verschieblichen starren Linsen (=passiv-optisches Element), hat die Aufgabe, das Abbildungsverhalten im Strahlengang zu beeinflussen. Es muss im sichtbaren Wellenlängenbereich transparent sein und muss die Lage und / oder die Form mindestens einer seiner lichtbrechenden Grenzflächen zeitlich ändern können, um die Scheitelbrechkraft des dioptrischen Apparates zu verändern. Die aktorische Komponente besteht dabei aus Energiestellern und Energiewandlern (Grote / Feldhusen (Hrsg.): Dubbel - Taschenbuch für den Maschinenbau. 21. Auflage. Springer Verlag Berlin Heidelberg New York (2005)), welche unter Einwirkung von Stellsignalen einer informationsverarbeitenden Einrichtung Kräfte realisiert, die dann in Bewegung umgesetzt werden können.

Im Falle eines passiv-optischen Elementes werden eine oder mehrere starre Linsen von einem Aktor axial im Strahlengang verschoben. Dieses Wirkprinzip wird standardgemäß in technischen Produkten zur Fokussierung eingesetzt. DE4300840A1 beschreibt z.B. ein Varioobjektiv für Kompaktkameras, das aus zwei Linsengruppen besteht, deren Abstand relativ zueinander variiert werden kann, um eine Brennweitenverstellung durchzuführen.

Verschiedene Mechanismen können zur Erfüllung der oben beschriebenen Aufgabe eines aktiv-optischen Elements zum Einsatz kommen. Dabei ist zwischen einer Änderung der Brechungsindexverteilung und einer Krümmungsänderung einer zwei Medien unterschiedlicher Brechungsindizes trennenden Grenzfläche zu unterscheiden. Diese Veränderungen können durch verschiedene physikalische Wirkungsprinzipien realisiert werden, die im Folgenden dargestellt werden.

Brechungsindexänderung durch elektrooptische Materialien: Die doppelbrechende Eigenschaft elektrooptischer Materialien kann durch elektromagnetische Felder beeinflusst werden. Dadurch kann eine definierte Brechungsindexverteilung eingestellt werden, die eine gezielte Beeinflussung des Abbildungsverhaltens in einer Polarisationsebene des Lichts ermöglicht. Diese kann neben einer gezielten Änderung der Fokuslage auch die Korrektur von Abbildungsfehlern höherer Ordnung (z.B. Astigmatismen, sphärische Aberration, Koma) umfassen. Um beide zueinander senkrechten Polarisationsebenen gleichermaßen zu beeinflussen, ist eine rechtwinklig gekreuzte Hintereinanderanordnung zweier derartiger Systeme notwendig. In der US6619799 wird die Verwendung eines derartigen aktiv-optischen Elementes in einem Brillengestell beschrieben. Dabei ist die elektrooptische Schicht von zwei transparenten Elektrodenflächen umfasst, zwischen denen eine elektrische Spannung angelegt werden kann, um das radiale Brechungsindexprofil zu verändern. Ein gewünschtes Brechungsindexprofil kann entweder durch Amplituden- und Frequenzmodulation der Steuerspannung oder durch Aufteilung der Elektroden in mehrere Bereiche, die mit jeweils unterschiedlichen Spannungen versorgt werden, erreicht werden.

Brechungsindexänderung durch Dichteänderung eines kompressiblen Fluids: Der Brechungsindex eines kompressiblen Fluids (z.B. eines Gases oder Gasgemisches) ist von der Dichte abhängig. Diese Abhängigkeit wird durch die Gladstone-Dale-Konstante beschrieben. Werden in einer gasgefüllten Kammer, die ein oder mehrere gekrümmte Begrenzungsflächen aufweist, der Druck und / oder die Temperatur geändert, ändert sich demnach auch das Abbildungsverhalten des optischen Systems. US4732458 beschreibt z.B. eine derartige Anordnung für ein Mehrlinsenelement, dessen Brechkraft kontinuierlich verändert werden kann. Die Druckerhöhung in der starren gasgefüllten Kammer wird durch einen in einem Zylinder geführten verschieblichen Kolben, der außerhalb der optischen Achse angeordnet ist, realisiert.

Geometrieänderung durch äußere Krafteinwirkung auf einen elastischen Festkörper: Ein elastischer Festkörper, dessen Brechungsindex sich von dem der Umgebung unterscheidet, kann durch Einwirkung äußerer Kräfte so verformt werden, dass sich die Krümmung seiner lichtbrechenden Oberflächen ändert und dadurch das optische Abbildungsverhalten beeinflusst wird. US6493151 beschreibt z.B. eine Anordnung für einen derartig verformbaren homogen oder inhomogen aufgebauten Festkörper, auf den durch einen in seinem Durchmesser veränderbaren Ring radiale Kräfte übertragen werden können. Die Durchmesseränderung des Rings kann thermisch, oder durch magnetische / elektrische Felder erfolgen. DE4345070 beschreibt beispielsweise eine Anordnung für einen verformbaren hüllenförmigen Festkörper, der mit einer lichtdurchlässigen Flüssigkeit gefüllt ist und dessen lichtbrechende Oberflächen über einen ringförmigen Fluidaktor hydraulisch oder pneumatisch verformt werden. DE10244312 nennt als Anwendungsbeispiel für einen Aktor aus Buckypaper (papierartiges Netzwerk von Kohlenstoffnanoröhren) die Änderung der Brechkraft einer künstlichen, in den Augapfel implantierten deformierbaren Linse.

Geometrieänderung durch Benetzungswinkelbeeinflussung (Electrowetting): Zwei ineinander nicht mischbare Fluide annähernd gleicher Dichte, die sich in ihren Brechungsindizes unterscheiden, bilden eine sphärisch gekrümmte oder plane Grenzfläche (Meniskus). Wird das eine, elektrisch leitfähige Fluid, in Kontakt mit einer Elektrode gebracht und gegenüber einer zweiten, von den beiden Fluiden durch eine isolierende Schicht (Dielektrikum) getrennte Elektrode eine Potentialdifferenz angelegt, so lässt sich der Benetzungswinkel und somit die Krümmung des Meniskus durch den sog. Elektrowetting-Effekt ändern. Da der Meniskus zwei Medien unterschiedlichen Brechungsindex trennt, wird das optische Abbildungsverhalten verändert. WO99/18456 beschreibt eine axiale Anordnung von leitfähigem Fluid, transparentem Dielektrikum und transparenter Elektrode im Strahlengang und Maßnahmen zur radialen Zentrierung des Tropfens in der optischen Achse. WO03/069380 beschreibt eine Anordnung, bei der die mit einem Dielektrikum beschichtete Elektrode zylindrisch um die optische Achse angeordnet ist. In der optischen Achse befinden sich axial hintereinander angeordnet das elektrisch-leitfähige Fluid und das isolierende Fluid, sowie der die beiden trennende Meniskus.

Geometrieänderung durch Druckänderung eines Fluides: Wird in einer fluidbefüllten Kammer, die eine oder mehrere deformierbare Begrenzungsflächen aufweist, die Druckdifferenz zur Umgebung geändert, kommt es zu einer Krümmungsänderung der Begrenzungsflächen und demnach auch zur Änderung des Abbildungsverhaltens des optischen Systems. US4466706 beschreibt beispielsweise eine derartige Anordnung, wobei die Druckdifferenzänderung durch einen Verdrängungsmechanismus erreicht wird. Dabei wird durch Drehung einer sich in der zylindrischen Ummantelung befindliche Schraube Fluid verdrängt, was zur Krümmungsänderung der beiden Zylinderendflächen führt. Alternativ kann die zylindrische Ummantelung auch zweiteilig ausgeführt sein, wobei eine Verdrängungswirkung durch eine axiale Relativbewegung beider Teile erzielt werden kann.

Geometrieänderung durch Kraftentwicklung innerhalb eines intelligenten Materials: Intelligente Materialien (Smart Materials) können durch Änderungen ihrer atomaren / molekularen Struktur Kräfte entwickeln und sich dadurch verformen. Durch die Einstellung eines Grenzflächenprofils zwischen intelligentem Material und Umgebung lässt sich demzufolge ebenfalls das optische Abbildungsverhalten beeinflussen. US2004/0100704 beschreibt z.B. einen zu diesem Zwecke verwendeten Formgedächtniskunststoff, der als Phase oder Schicht innerhalb eines verformbaren Linsenkörpers eingebracht ist und die Form des Körpers unter Einwirkung von Energie lokal verändern kann. Als Anwendungsbeispiel wird die postoperative, nicht reversible Korrektur des Abbildungsverhaltens implantierter Intraokularlinsen genannt. JP01230004 beschreibt beispielsweise die Verwendung eines schwellbaren Gels und eines Lösungsmittels, die innerhalb eines deformierbaren Festkörpers schichtartig angeordnet sind. Unter Einwirkung einer Spannung kann die Löslichkeit des Lösungsmittels im schwellbaren Gel so verändert werden, dass dieses daraufhin eine Volumenänderung erfährt. Diese bewirkt eine Krümmungsänderung der lichtbrechenden Oberfläche.

Auch Kombinationen der genannten Wirkprinzipien sind möglich. Das optische System ist folglich in der Lage, die Fokuslage des dioptrischen Apparates anzupassen. Das optische System kann des Weiteren mehrere Sensorelemente umfassen, um das optische Abbildungsverhalten im Strahlengang zu optimieren. Ein enthaltenes aktivoptisches Element kann gegebenenfalls weitere Abbildungsfehler (monochromatische und chromatische Aberrationen) statisch oder auch dynamisch korrigieren (lokale Beeinflussung der Lichtwellenfront).

Zur Generierung der Stellsignale für die aktorische Komponente des aktiv-optischen Elementes bzw. des passiv-optischen Elementes, ist es notwendig, Informationen zu erfassen, aus denen auf die notwendige Scheitelbrechkrafterhöhung (=Akkommodationsbedarf) geschlossen werden kann.

Die Erfassung der Steuersignale der Pupillenweite allein reicht zur Bestimmung des Akkommodationsbedarfes nicht aus, da die Messgröße von zwei Einflussgrößen (der Leuchtdichte und der Objektdistanz) abhängt. Eine Möglichkeit zur Informationserfassung stellt die gleichzeitige Messung der Pupillenweite bzw. der Iriskontraktions/-dilatationsbewegung und der Leuchtdichte im Strahlengang dar, um die Pupillennahreaktion vom Pupillenlichtreflex zu trennen.

Die erfindungsgemäße Verwendung der Sensoren zur Detektion des Pupillendurchmessers, welche als Implantat verwendet werden, ermöglicht die Kombination der Messung von Umfeldleuchtdichte und Pupillendurchmesser.
Der Einsatz eines Sensors oder mehrerer Sensoren zur Messung des Pupillendurchmessers und der Umfeldleuchtdichte im Rahmen des einstückigen künstlichen Akkommodationssystems ist deshalb vorteilhaft, weil der Pupillendurchmesser monoton mit zunehmendem Akkommodationsbedarf (Pupillennahreflex) sowie mit zunehmender Umfeldleuchtdichte (Pupillenlichtreflex) sinkt. Um den Akkommodationsbedarf mit Hilfe des Pupillennahreflexes bestimmen zu können, ist daher die Messung des Pupillendurchmessers sowie die Messung der Umfeldleuchtdichte notwendig.

Die erfindungsgemäß einsetzbaren Sensoren können alle nach dem Stand der Technik geeigneten Vorrichtungen zur Messung von Pupillendurchmesser und Umfeldleuchtdichte enthalten. Jeder Sensor umfasst mindestens ein Sensorelement. Ein Sensorelement ist eine lichtempfindliche Fläche, z. B. Photodiode, Photowiderstand, Phototransistor, CMOS (Complementary Metal Oxide Semiconductor) oder CCD (Charged Coupled Device) -Sensorelement, die eingehende Photonen in eine analoges oder digitales Signal wandelt.

Bevorzugt werden mindestens zwei Sensorelemente eingesetzt, eines im Strahlengang ohne Abdeckung durch die Iris für die Lichtmessung (Umfeldleuchtdichte) und mindestens ein anderes, das durch die Iris im Bereich des Pupillenrandes teilweise oder ganz abgedeckt ist und über die Abschattung durch die Iris ein Signal zur Bestimmung die Pupillenweite erzeugt.

Vorzugsweise erfolgt eine lineare Aneinanderreihung der Sensorelemente zu einer Sensorelementzeile, deren Breite vorzugsweise 1 - 500 µm, besonders bevorzugt 10 - 50 µm beträgt. Die einzelnen Sensorelemente weisen in Reihenrichtung eine wesentlich kleinere Länge von bevorzugt 1 bis 15 µm auf. Die bevorzugten Maximalabmessungen eines Sensorelements betragen somit 500 x 15 µm.

Im Gegensatz zum Stand der Technik kann durch die Verwendung von Sensoren in der genannten Größe die beschriebene Messung von einem Implantat aus durchgeführt werden. Daher ist die Verwendung eines vorzugsweise einstückigen künstlichen Akkommodationssystem ohne eine taktile oder elektrische Anbindung des Gewebes möglich. Die Möglichkeit für ein einstückiges künstliches Akkommodationssystem mit nur einem Implantationsort, dem Kapselsack, vereinfacht die Implantation erheblich. Da das System ohne elektrische oder taktile Anbindung an den Körper messen kann, ist eine ausreichend genaue Messung unabhängig von eventuell auftretenden Gewebeveränderungen möglich.

Der Einsatz der Sensoren kann in mehreren Ausführungsvarianten erfolgen:
Eine kann darin bestehen, daß im Implantat hinter der Iris ein linear verteilter, aus mehreren Sensorelementen bestehender Sensor positioniert wird. Der betreffende Sensor ist in der Lage, die auf jedes Sensorelement fallende Beleuchtungsstärke zu detektieren. Dabei muß gewährleistet sein, daß mindestens ein Sensorlement, welches vorzugsweise zentral anzuordnen ist, unter keinen Umständen durch die Iris verdeckt wird. Mit Hilfe dieser Sensorelemente kann die Umfeldleuchtdichte ermittelt werden.

Die weiteren Teile sind derart zu verteilen, daß unabhängig vom Pupillendurchmesser, welcher beim Menschen zwischen 2 und 10 mm variiert, stets mindestens ein Sensorelement teilweise von der Pupille überdeckt wird oder eine bezüglich des Pupillennahreflexes vernachlässigbare Pupillendurchmesseränderung zu einem solchen Zustand führt. Sind diese Sensorelemente sehr klein, kann direkt aus der Anzahl der von der Pupille überdeckten und damit unbeleuchteten Sensorelemente auf den Pupillendurchmesser geschlossen werden. Bei größeren Sensorelementen kann anhand der Anzahl der überdeckten Teile und anhand des Verhältnisses der Beleuchtung des/der zentralen Teile (s) zu den teilweise beleuchteten Sensorelementen auf den Pupillendurchmesser geschlossen werden.

Vorzugsweise ist mindestens ein Sensorelement zur Messung der Umfeldleuchtdichte aus dem nicht durch die Iris verdeckten Strahlengang des Auges vorgesehen. In jeder Pupillenstellung ist der Pupillenrand und damit der Pupillendurchmesser durch mindestens ein Sensorelement detektierbar.

Zur Kompensation von Positionsfehlern sind in jeder Irisstellung drei Sensorelemente zur Bestimmung des Pupillendurchmessers an unterschiedlichen Stellen des Pupillenrandes vorzugsweise angeordnet. Ab mindestens drei solcher Elemente lässt sich der Pupillendurchmesser auch bei einer Dezentrierung des Implantats durch das Messsignal rekonstruieren. Vorzugsweise werden die nicht zentral liegenden Elemente auf Strecken angeordnet, die von der optischen Achse radial nach außen und rotationsymmetrisch liegen.

Optional besteht auch die Möglichkeit, dass durch die Pupille einfallende Licht auf Sensorelemente außerhalb des Strahlengangs umzulenken. Im Strahlengang können Mittel zur Umlenkung des einfallenden Lichts auf den außerhalb des Strahlengangs angeordneten Sensors vorgesehenen sein. Hierbei umfassen die Mittel ein Strahlen umlenkendes oder -führendes Element im Strahlengang durch die Pupille. Das betreffende Strahlen umlenkende oder -führende Element umfasst hierbei vorzugsweise eine Reflektionsebene. In diesem Fall können außerhalb des Strahlengangs auch größere Sensoren verwendet werden, die im Strahlengang als störend wahrgenommen werden. Mit den beschriebenen Sensoren ist die Messung von Pupillendurchmesser und Umfeldleuchtdichte von einem Implantat aus möglich. Das heißt, erfindungsgemäß kann eine Verwendung im Rahmen eines einstückigen künstlichen Akkommodationssystem erfolgen.

Die erfassten Informationen werden im Rahmen der hier beschriebenen Erfindung dem Informationsverarbeitungssystem zur Verfügung gestellt. Gegenstand der Erfindung ist aber auch ein oben beschriebenes Informationserfassungssystem allein, welches Messdaten zur Registrierung und Weiterverarbeitung an einen Empfänger außerhalb des Körpers senden kann.

Die erfassten Signale werden vom Informationsverarbeitungssystem aufbereitet (z.B. Ausreißertests, Glättung, Filterung, Verstärkung). Mit Methoden der klassischen Statistik, der Computational Intelligence und Data Mining werden Merkmale extrahiert und klassifiziert, um die Akkommodationsabsicht zu detektieren. Mit Hilfe von steuerungs- und regelungstechnischen Methoden (z.B. fuzzy-gesteuerter PID-Regler, adaptive Regelungsalgorithmen, selbstlernende Algorithmen) werden die benötigten Stellsignale für das optische System generiert. Es können sowohl hierarchische Regelungsstrukturen als auch zentral-dezentrale Strukturen zum Einsatz kommen.

Zur Versorgung der Subsysteme mit Energie wird ein Energieversorgungssystem eingesetzt, das aus einem Energiewandler, einem Energiespeicher und einer Steuerungseinheit bestehen kann. Der Energiewandler transformiert von außen fernübertragene Energie (z.B. induktiv, kapazitiv, optisch) oder gespeicherte Energie (z.B. Batterie, Miniatur-Brennstoffzelle), die auch in Form von Körperflüssigkeiten (z.B. das nährstoffreiche Kammerwasser, Blut) vorliegen kann, bzw. mechanische Energie (z.B. aus Muskelbewegungen) über einen Energiespeicher in elektrische Energie. Diese wird durch die Steuereinheit des Energieversorgungssystems zu genau definierten Zeitpunkten an die Subsysteme abgegeben.

Durch den Vergleich der mit diesem Sensor gemessenen Beleuchtungsstärke mit einem Schwellwert, kann der Energieverbrauch des Gesamtsystems in Zuständen, in denen keine Akkommodationsfähigkeit notwendig ist, reduziert werden. D. h. das System besitzt eine Vorrichtung zur Umschaltung in den energieverbrauchsarmen Stand-by-Zustand bei Unterschreitung eines Schwellwertes der Leuchtdichte. Bei Wiederüberschreiten des Schwellwertes erfolgt die Umschaltung in den Betriebszustand.

Das Gesamtsystem wird mit Hilfe von geeigneten Befestigungselementen zur axialen Fixierung und radialen Zentrierung im Strahlengang implantiert. Aus der Ophthalmologie (Augenheilkunde) sind für Intraokularlinsen zahlreiche Haptikausführungen bekannt. (Draeger, J.; Guthoff, R.F.: Kunstlinsenimplantation. In: Augenheilkunde in Klinik und Praxis Band 4. Hrsg.: Francois, J.; Hollwich, F. Georg Thieme Verlag Stuttgart New York (1991); Auffarth, G.U.; Apple, D.J.: Zur Entwicklungsgeschichte der Intraokularlinsen. Ophthalmologe 98(11) (2001) 1017-1028). Diese können vorzugsweise im Kammerwinkel, im Sulcus ciliaris oder im Kapselsack Halt finden.

Das künstliche Akkommodationssystem ist der technische Teil eines Regelungssystems (geschlossener Regelkreis), welches als künstliches System die Funktion der natürlichen verformbaren Augenlinse und des Ziliarmuskels eines Patienten substituiert. Der biologische Teil besteht im Wesentlichen aus: der Hornhaut, dem Kammerwasser und dem Glaskörper als Bestandteile des dioptrischen Apparates, der Netzhaut als natürlichem Sensorarray und dem Gehirn als natürliche Informationsverarbeitungseinheit, die Steuersignale erzeugt, die Informationen über den Akkommodationsbedarf enthalten.

Das künstliche Akkommodationssystem umfasst ein optisches System, mit einer verstellbaren Brennweite und/oder anderen optischen Eigenschaften. Dieses bildet einen neu eingebrachten Bestandteil des dioptrischen Apparates des Patienten. Es umfasst ein Informationserfassungssystem, das optisch die Pupillenweite und Leuchtdichte erfasst. Auf Basis dieser Messungen wird der Akkommodationsbedarf durch ein Informationsverarbeitungssystem ermittelt und es werden Stellsignale zum Ansteuern des optischen Systems generiert. Das System wird über ein geeignetes Energieversorgungssystem gespeist und ist über ein Befestigungssystem im Patientenauge fixiert.

Das beschriebene Akkommodationssystem kann zur Wiederherstellung der Akkommodationsfähigkeit nach Entfernung der natürlichen Augenlinse bei Linsentrübung (Katarakt) oder Alterssichtigkeit (Presbyopie) dienen.

Im Folgenden wird die Erfindung unter Bezugnahme auf die Figuren näher beschrieben:

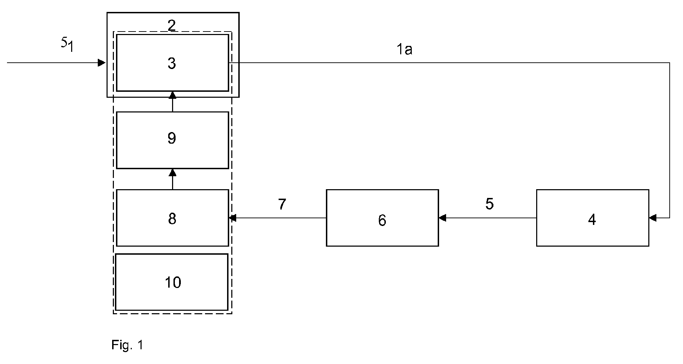

In Figur 1 ist eine schematische Darstellung des Gesamtsystems (künstliches Akkommodationssystem) wiedergegeben. Die Information 1, z. B. Licht von einem Objekt in zeitlich veränderlicher Gegenstandsweite fällt durch den dioptrischen Apparat des menschlichen Auges 2, der das optische System 3 enthält. Das fokussierte Licht 1 a fällt auf den natürlichen Sensor, die Netzhaut 4.

Die durch die Photorezeptoren generierten afferenten Signale 5 werden dem natürlichen Informationsverarbeitungssystem 6, dem Gehirn, zugeleitet. Von dort werden efferente Signale 7, die Information über den Akkommodationsbedarf enthalten, an motorische Strukturen (z. B. Irismuskel, Ziliarmuskeln, Bulbusmuskeln) gesendet. Diese Information wird vom Informationserfassungssystem 8 des künstlichen Akkommodationssystems aufgenommen. Das Informationsverarbeitungssystem 9 leitet daraus Stellsignale für das optische System 3 ab. Damit wird die Scheitelberechkraft des dioptrischen Apparates 2 durch das künstliche Akkommodationssystem an den Akkommodationsbedarf angepaßt, der aus zeitlich veränderlichen Gegenstandsweiten resultiert. 10 stellt das Energieversorungssystem dar. Alle technischen Systembestandteile sind durch eine gestrichelte Linie eingerahmt.

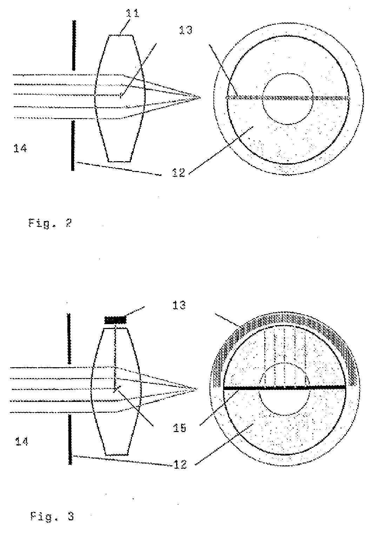

In Figur 2 ist eine schematische Darstellung einer Möglichkeit einer Anwendung der erfindungsgemäßen Sensoren beschrieben. Hierbei ist der Sensor 13 zentral im Implantat 11 hinter der Iris 12 positioniert. Mit Hilfe des Sensors 13 kann die einfallende Strahlung 14 gemessen werden. Um eine Bestimmung der Umfeldleuchtdichte erreichen zu können ist es unbedingt erforderlich, dass mindestens ein Sensorelement nicht durch die Iris verdeckt wird. Hierbei ist das Sensorelement vorzugsweise zentral anzuordnen.

Die weiteren Teile sind dagegen so angeordnet, daß wenigstens ein Sensorlement genau teilweise von der Pupille überdeckt wird. Die Größe der Sensorelemente wird vorzugsweise so klein gewählt, daß direkt aus der Anzahl der von der Pupille überdeckten und damit unbeleuchteten Sensorelemente auf den Pupillendurchmesser geschlossen werden. Jedoch ist auch der Einsatz größerer Sensorelemente möglich. Hier kann anhand der Anzahl der überdeckten Teile und anhand des Verhältnisses der Gesamtbeleuchtung zu den teilweise beleuchteten Sensorlementen auf den Pupillendurchmesser geschlossen werden.

In Figur 3 ist eine Anwendungsvariante dargestellt. Danach besteht die Möglichkeit mit Spiegeln 15, das durch die Pupille einfallende Licht 14 auf Sensorelemente 13 außerhalb des Strahlengangs umzulenken. In diesem Fall lassen sich vorteilhafterweise auch größere Sensoren einsetzen, die ansonsten im Strahlengang als störend wahrgenommen würden.

## Patentansprüche

1. Implantierbares Akkomodationssystem zur Bestimmung des Akkomodationsbedarfs und zur Wiederherstellung der Akkomodationsfähigkeit durch optische Messung des Pupillendurchmessers und der Umfelddichte bei welchem
a) wenigstens ein optisches System aus einem oder mehreren aktiv-optischen Elementen und/oder eine oder mehreren von Aktoren achsial verschieblichen starren Linsen
b) wenigstens ein zum Ziliarmuskel berührungsloses Informationserfassungssystem mit Mitteln zur Messung einer Pupillenweite und einer Leuchtdichte mindestens an einem Auge als körpereigenes Signal für den Akkomodationsbedarf
c) wenigstens ein Informationsverarbeitungssystem, in welchem die erfassten Signale aufbereitet und die für das optische System benötigten Stellsignale generiert werden,
d) wenigstens ein Energieversorgungssystem und wenigstens ein Befestigungssystem zu einem Implantat zusammengefasst sind, wobei
das System einen Sensor oder mehrere Sensoren mit Sensorelementen zur optischen Messung des Pupillendurchmessers und der Umfeldleuchtdichte aufweist, wobei im Implantat hinter der Iris ein linear verteilter, aus mehreren Sensorelementen bestehender Sensor positionierbar ist.

2. System nach Anspruch 1,
**dadurch gekennzeichnet, dass** die Sensorelemente photosensitive Sensorelemente enthalten.

3. System nach Anspruch 2,
**dadurch gekennzeichnet, dass** die photosensitiven Sensorelemente Fotodioden, Fotowiderstände, Fototransistoren, CCD oder CMOS-Sensorelemente sind.

4. System nach einem der vorgenannten Ansprüche, **dadurch gekennzeichnet, dass** mindestens ein Sensorelement zur Messung der Umfeldleuchtdichte aus dem nicht durch die Iris verdeckten Strahlengang des Auges vorgesehen ist und in jeder Pupillenstellung der Pupillenrand und damit der Pupillendurchmesser durch mindestens ein Sensorelement detektierbar ist.

5. System nach Anspruch 4, **dadurch gekennzeichnet, dass** in jeder Irisstellung drei Sensorelemente zur Bestimmung des Pupillendurchmessers an unterschiedlichen Stellen des Pupillenrandes angeordnet sind.

6. System nach einem der vorgenannten Ansprüche, **dadurch gekennzeichnet, dass** die Sensorelemente zu mindestens einer Sensorelementzeile aneinandergereiht zu mindestens einem Sensor zusammengesetzt sind.

7. System nach Anspruch 6, **dadurch gekennzeichnet, dass** die Sensorelementzeile jeweils eine Zeilenhöhe von 1 - 500 Mikrometern
aufweisen.

8. System nach einem der vorgenannten Ansprüche, **dadurch gekennzeichnet, dass** der Sensor im Strahlengang durch die Pupille angeordnet ist.

9. System nach einem der vorgenannten Ansprüche, **dadurch gekennzeichnet, dass** im Strahlengang Mittel zur Umlenkung des einfallendes Lichts auf den außerhalb des Strahlengangs angeordneten Sensors vorgesehen sind.

10. System nach Anspruch 9, **dadurch gekennzeichnet, dass** die Mittel ein strahlenumlenkendes oder -führendes Element im Strahlengang durch die Pupille umfassen.

11. System nach Anspruch 10, **dadurch gekennzeichnet, dass** das strahlenumlenkende oder -führende Element eine Reflexionsebene umfasst.

12. System nach einem der Ansprüche 8 bis 11, **dadurch gekennzeichnet, dass** der Sensor oder das strahlenumlenkende oder -führende Element im optischen System integriert ist.

## Claims

1. An implantable accomodation system for determining the accommodation requirement and restoration of the accomodation ability by optical measurement of the pupil diameter and the surrounding luminance, comprising in that
a) at least one optical system comprising one or more active-optical elements and/or one or more inflexible lenses that are axially adjustable by one or a plurality of actors,
b) at least one data acquisition system which does not contact the ciliary muscle and which is provided with means for measuring a diameter of the pupil and the luminance around at least one eye as a physical control signal for the accommodation requirement,
c) at least one data processing system for processing the recorded signals and for generating actuatings necessary for the optical system,
d) at least one energy supply system and at least one fixing system are combined as an implant,
**characterized**
**in that** the system has one sensor or a plurality of sensors with sensor elements for the optical measuring of the pupil diameter and the surrounding luminance, whereby in the implant behind the iris a linearly distributed sensor can be positioned which consists of a plurality of sensor elements.

2. The system as claimed in claim 1,
**characterized in that** the sensor elements comprise photosensitive sensor elements.

3. The system as claimed in claim 2,
**characterized in that** the photosensitive sensor elements are photodiodes, photoresistors, phototransistors, CCD or CMOS sensor elements.

4. The system as claimed in one of the preceding claims, **characterized in that** at least one sensor element is provided for measuring the surrounding luminance from the beam path of the eye not covered by the iris and the pupil edge and hence the pupil diameter can be detected by at least one sensor element in every pupil position.

5. The system as claimed in claim 4, **characterized in that** three sensor elements for determining the pupil diameter are arranged at different locations of the pupil edge in every iris position.

6. The system as claimed in one of the preceding claims, **characterized in that** the sensor elements, arranged next to one another to form at least one sensor element row, are composed to form at least one sensor.

7. The system as claimed in claim 6, **characterized in that** the sensor element rows in each case have a row height of 1-500 micrometers.

8. The system as claimed in one of the preceding claims, **characterized in that** the sensor is arranged in the beam path through the pupil.

9. The system as claimed in one of the preceding claims, **characterized in that** means are provided in the beam path for deflecting the incident light onto the sensor arranged outside of the beam path.

10. The system as claimed in claim 9, **characterized in that** the means comprise a beam deflecting or beam guiding element in the beam path through the pupil.

11. The system as claimed in claim 10, **characterized in that** the beam deflecting or beam guiding element comprises a reflection plane.

12. The system as claimed in one of claims 8 to 11, **characterized in that** the senor or the beam deflecting or beam guiding element is integrated into the optical system.

## Revendications

1. Système d'accommodation implantable destiné à déterminer le besoin d'accommodation et à rétablir la capacité d'accommodation par mesure optique du diamètre de pupille et de la luminance ambiante, dans lequel
a) au moins un système optique constitué d'un ou de plusieurs éléments optiques actifs et/ou d'une ou plusieurs lentilles rigides pouvant être déplacées axialement par des actionneurs,
b) au moins un système de détection sans contact d'informations concernant les muscles ciliaires, comportant des moyens destinés à mesurer une largeur pupillaire et une luminance au moins au niveau d'un oeil en tant que signal corporel indiquant le besoin d'accommodation,
c) au moins un système de traitement d'informations dans lequel les signaux détectés sont traités et les signaux de réglage nécessaires au système optique sont générés,
d) au moins un système d'alimentation en énergie et au moins un système de fixation sont assemblés pour former un implant, dans lequel
le système comprend un capteur ou plusieurs capteurs ayant des éléments de capteur destinés à la mesure optique du diamètre de pupille et de la luminance ambiante, dans lequel un capteur constitué d'une pluralité d'éléments de capteur divisés linéaires peut être positionné à l'arrière de l'iris.

2. Système selon la revendication 1,
**caractérisé en ce que** les éléments de capteur comportent des éléments de capteur photosensibles.

3. Système selon la revendication 2,
**caractérisé en ce que** les éléments de capteur photosensibles sont des photodiodes, des photorésistances, des phototransistors, des éléments de capteur à CCD ou CMOS.

4. Système selon l'une quelconque des revendications précédentes,
**caractérisé en ce qu'**au moins un élément de capteur est prévu pour mesurer la luminance ambiante à partir du chemin optique de l'oeil non recouvert par l'iris et **en ce qu'**ainsi et à chaque position de la pupille, le bord de la pupille et par conséquent, le diamètre de pupille peuvent être détectés par au moins un élément de capteur.

5. Système selon la revendication 4, **caractérisé en ce qu'**à chaque position de l'iris, trois éléments de capteur sont disposés en des points différents du bord de la pupille pour déterminer le diamètre de pupille.

6. Système selon l'une quelconque des revendications précédentes, **caractérisé en ce que** les éléments de capteur sont assemblés en rangées sous la forme d'au moins une ligne d'éléments de capteur en au moins un capteur.

7. Système selon la revendication 6, **caractérisé en ce que** les lignes d'éléments de capteur présentent chacune une hauteur de ligne de 1-500 micromètres.

8. Système selon l'une quelconque des revendications précédentes, **caractérisé en ce que** le capteur est disposé sur le chemin optique passant à travers la pupille.

9. Système selon l'une quelconque des revendications précédentes, **caractérisé en ce qu'**il est prévu sur le chemin optique des moyens destinés à dévier la lumière incidente sur le capteur disposé en dehors du chemin optique.

10. Système selon la revendication 9, **caractérisé en ce que** les moyens comprennent un élément de déviation ou de guidage de faisceau sur le chemin optique passant à travers la pupille.

11. Système selon la revendication 10, **caractérisé en ce que** l'élément de déviation ou de guidage de faisceau comprend un plan de réflexion.

12. Système selon l'une quelconque des revendications 8 à 11, **caractérisé en ce que** le capteur ou l'élément de déviation ou de guidage de faisceau est intégré au système optique.
